# EUROPEAN PATENT SPECIFICATION

(11) **EP 2 201 364 B1**
(45) Date of publication and mention of the grant of the patent: **17.08.2016**
(21) Application number: 08835602.7
(22) Date of filing: 11.09.2008
(51) Int. Cl.: G01N 33/48, A61B 5/06, A61B 8/08, G01N 21/65, G01N 29/12, A61B 1/06, G01N 21/47, G01N 29/24, A61B 5/00

(54) **ARRANGEMENT FOR UNAFFECTED MATERIAL ANALYSIS**
ANORDNUNG ZUR UNBEEINFLUSSTEN MATERIALANALYSE
DISPOSITIF POUR UNE ANALYSE SANS EFFET SUR LE MATÉRIAU

(30) Priority: 01.10.2007 SE 0702207
(43) Date of publication of application: 30.06.2010
(73) Proprietor: Medsens AB, 907 19 Umeå (SE)
(72) Inventor: RAMSER, Kerstin, S-973 33 Luleå (SE); LINDAHL, Olof, S-906 51 Umeå (SE)
(74) Representative: Zacco Sweden AB
(86) International application number: PCT/SE2008/051018
(87) International publication number: WO 2009/045152

(56) References cited:
- EP-A2- 0 764 842
- WO-A2-2006/121877
- FR-A1- 2 869 521
- US-A1- 2003 135 118
- US-A1- 2004 152 992
- US-A1- 2007 032 747
- US-B1- 6 205 354

## Description

This invention concerns an arrangement for unaffected material analyse.

It is desirable and necessary within many technical areas to investigate material and its properties, in order, for example, to detect, localise and determine at least one of changes, deviations, variations, differences, defects and similar in the material and to check the distributions of material, both desired and undesired.

Such material analysis has previously occurred through the material being cut and visually investigated, or through parts of the material being removed, samples of the material having been taken, and investigated in, for example, a microscope. This mechanical processing that occurs during such a material sampling normally destroys the material in question, at least in that part of it from which the sample is taken, or it causes or initiates other types of material change, or risks that undesired material changes take place.

The material may be a manufactured material or a natural material. The material may be an inorganic material or an organic material, such as living tissue.

US 2007/032747 discloses a device for tissue characterization comprising several types of sensors. Tactile and spectral sensors are among these sensors.

One purpose of this invention is to offer an arrangement that make it possible to carry out a unaffected material analyse for the detection and analysis of at least one material deviation, variation, difference or similar, in a material.

This purpose is achieved with an arrangement having the technical characteristics that are made clear by claims 1.

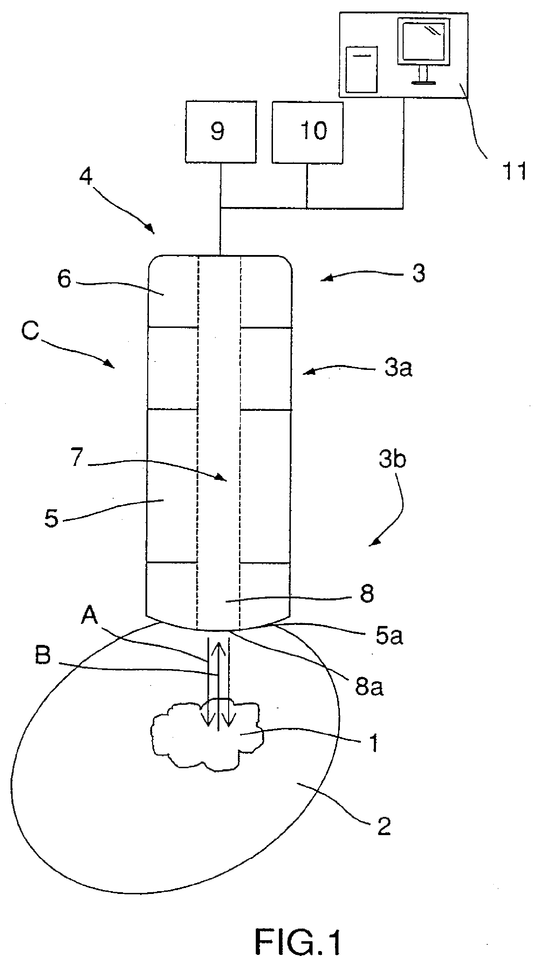

The invention will be described here with reference to the attached drawing. Figure 1 shows a schematic figure of an arrangement according to the invention.

The basic idea of the invention is to make it possible to combine the detection of a harder or softer region 1 in a material 2 with a material analysis of the detected region that takes place directly. This is to be carried out directly in the material without the need for any form of sampling, without the need that any material be removed. This leads to the influence on the material being kept to a minimum and it makes it possible to avoid future changes in the material as a result of the removal of material. This will be referred to below as "unaffected material analyse". It is also a basic idea that it should be possible to carry out this method with the aid of one and the same arrangement 3.

A method not being part of the scope of the invention is intended to be used during unaffected material analyse for the detection and analysis of at least one region 1 that has a different hardness than that of the material otherwise, a material deviation, in a material 2 for which the basic properties, the desired properties, are known.

The method comprises the detection of a region 1 with a different hardness, stiffness, than the known material, a region 1 with a difference in hardness from that of the material otherwise. The detection of the region 1 takes place through the use of a tactile sensor 4, which is placed in contact with the material 2. The tactile sensor 4 reads differences in hardness, and these differences are recorded and analysed. This may take place either manually or by machine; it may be activated or automatic; there may be different recording systems, processing systems, feedback systems, reporting systems and similar used; and the arrangements associated with these may be different.

When a region 1 with a deviating hardness, stiffness, is detected, the detected region 1 is illuminated with chromatographic light A. The energy of the chromatographic light is partially transferred to the material in the detected region 1. The chromatographic light that is reflected from the region 1 has transferred energy to the material in the region 1. A light spectrum is obtained by recording the change in energy.

The method comprises also the analysis of the light spectrum that has been obtained in order to obtain information concerning the physical and chemical molecular structure of the material that is present in the detected region. The reflection will demonstrate a specific appearance, a specific light spectrum, that depends on the identity of the material.

The analysis may take place either manually, by man, or by machine; it may be either activated or automatic. Depending on the technical area in which the method is being used, it is appropriate that the light spectrum that has been obtained be compared with light spectra from common substances and from substances within the technical area.

The detection of the region 1 with a different hardness takes place by causing contact to be made with the tactile sensor 4, which is appropriately a resonance sensor. The main component of a resonance sensor is an element 5, a piezoelectric element, a ceramic, which is caused to vibrate, to oscillate, approximately in the same way as a vibrating guitar string, with the aid of an electrical circuit. The element 5 vibrates with a particular frequency, its resonance frequency. When a load, for example a material 2, is subsequently placed in contact with one end of the vibrating element 5, the acoustic impedance of the material will influence the vibrating element, the oscillating system, such that it vibrates at a new resonance frequency. The frequency at which the element 5 vibrates is determined by the hardness, the stiffness, of the material with which the sensor is in contact. If the sensor 4 is moved across a material 2, continuous changes in frequency can be recorded, and one or several regions 1 with different hardnesses can be detected and localised. The frequency changes, the differences in frequency, that are recorded, registred, depend on the hardness, the stiffness, of the material with which the sensor is in contact.

Thus, the method comprises the activation of an electrical circuit 6 in order to cause the element 5, comprised within the resonance sensor 4, to vibrate in order to obtain a resonance frequency, and the placing of the element 4 in contact with the material 2, after which a difference in resonance frequency of the element can be measured and correlated with the hardness of the material with which the sensor is in contact, whereby a region 1 with a different hardness can be detected.

The method comprises the use of laser light in order to obtain the chromatographic light. It is appropriate and advantageous to use a Raman spectrometer 7, a Raman sensor, with a probe 8 that is placed in contact with the material. The Raman spectrometer 7 comprises a source 9 of laser light for illumination, and arrangements and units for the analysis. The use of such a sensor has proved to be valuable in the detection of cancerous changes in tissues. Tumours can be revealed since these usually demonstrate another chemical composition than that of healthy tissue, such as prostate tissue. Tumours give rise to a changed molecular composition of the tissue, and this is reflected in the spectrum that is recorded by the Raman spectrometer 7.

An arrangement 3 according to the invention is an arrangement 3 that makes unaffected material analyse possible, and that it is possible to use for the method. The arrangement comprises a tactile sensor 4 that is placed in contact with the material 2 for the detection of at least one region 1 with a difference in hardness compared with the known material 2. The tactile sensor 4 is a resonance sensor comprising an element 5 and an electrical circuit that is activated and that causes the element to vibrate in order to obtain a resonance frequency. The element 5 is a piezoelectric element.

The arrangement 3 comprises further an arrangement 9, a source of laser light, that emits chromatographic light and that illuminates the detected region 1 with the light A in order to receive reflected light B that can be reproduced as a light spectrum, and finally an arrangement 10 that comprises a detector (not shown in the drawing) and that analyses the light spectrum that has been received and that gives information about the material in the region, its physical and chemical molecular structure.

It is appropriate that the arrangement 3 comprise a Raman spectrometer 7, which in turn comprises a probe 8 that constitutes the end of an optical fibre through which the laser light propagates, the source 9 of laser light and the arrangement 10 for analysis and information processing. The arrangement 10 for analysis and information processing may be more or less manual or machine-based; it may be activated or automatic.

This invention, both the method and the arrangement, have principally been developed and tested within the technical area of medicine, where the unaffected material analyse has been carried out on living tissue, for the diagnosis of tumours, cancer tumours, in tissue, in prostate glands *in vivo.*

Histopathology is a common method for demonstrating the presence of cancer in tissue. It involves the detection and confirmation of the presence of morbid tissue changes *in vitro*, outside of the living organism, normally with a microscope. Histopathology is a time-consuming method that requires skilled personnel who are able to carry out correct sampling, normally in the form of a biopsy in which a tissue sample is taken with the aid of an instrument that is introduced into the tissue, and who are able to investigate, analyse and evaluate the sample, and interpret the result correctly.

It may be problematical to use this known method in certain situations, for example in cases of prostate cancer, which is the most common form of cancer affecting men in the USA and Europe. It is often not possible to locate tumours in the prostate with palpitation, analyse using the hands, nor with any commercially available imaging method, such as ultrasound, and this makes further sampling and analysis both uncertain and problematical.

Palpitation of the prostate takes place through a physician investigating the hardness of the tissue of the prostate using the fingers, *via* the rectum of the patient. The physician is seeking harder areas, since it is normally the case that tumours are harder than the surrounding healthy tissue. Even if the physician can feel harder areas and suspects the presence of cancer, it is difficult to determine the exact location of the tumour in the prostate.

In order to determine the location of any possible cancer tumour, it is necessary to take biopsies from several random locations in the prostate in order to obtain a clearer image of the location and extent of the cancer. The taking of biopsies entails creating wounds in skin and tissue and this increases the risk of complications arising, for example in the form of infections, since this type of sampling takes place in an area rich in bacteria close to the anus. It is also difficult to carry out the final analysis of the sample that has been taken. It has proved to be the case that cancer that is present is relatively often not detected. It has been estimated that this occurs as often as for 3 out of every 10 biopsies carried out.

The fact that the histopathologic analyses are carried out *in vitro* and cannot be carried out *in vivo*, in the living organism, since the sample that is to be analysed must be removed from the body, entails further risks for erroneous assessment of the sample of material, and of the disease condition related to it, since there is a risk that the sample is subjected to both mechanical and chemical changes during its removal from the body.

The main component of a resonance sensor is an element, a piezoelectric element, a ceramic, which can be caused to vibrate, approximately in the same way as a vibrating guitar string, with the aid of an electric circuit. The element vibrates with a particular frequency, its resonance frequency. When a load, for example a material, is subsequently placed in contact with one end of the vibrating element, the acoustic impedance of the material will influence the oscillating system such that it vibrates at a new resonance frequency. The frequency at which the element vibrates is determined by the hardness, the stiffness, of the material with which the sensor is in contact. If the sensor is moved across a material, continuous changes in frequency can be recorded, and one or several regions with different hardnesses can be detected and localised. The frequency changes, the differences in frequency, that are recorded, depend on the hardness, the stiffness, of the material with which the sensor is in contact.

The use of such a sensor has proved to be valuable in the detection of cancerous changes in tissues. Tumours can be revealed since these are usually harder than healthy tissue, such as prostate tissue.

Raman spectroscopy is a light-based method in which the material is illuminated with monochromatic light, normally laser light. The monochromatic light that impinges upon a material, a sample, causes motion in the illuminated molecules in the material and gives rise to changes in wavelength of the light, across the range of wavelengths that can be detected, and portions of the light are reflected back in the form of a spectrum, a spectrum of colours.

This spectrum is interpreted and provides detailed information about the molecular composition of the material and on the properties of the molecules.

An arrangement 3, an instrument, according to the invention combines two detection technologies in order not only to increase the diagnostic reliability but also to be able to provide supplementary information about which type of cell change is involved. The diagnosis will be better and more reliable since it is possible with one and the same arrangement to detect the presence of a material deviation, a tumour, and to analyse, investigate, it at the same time. Furthermore, the risk of infection, which is relatively common for sampling in which parts of the material, the tissue, are removed, is reduced.

The method and the arrangement 3 can be applied in other technical areas in which the components of a material have been accumulated to structures with a different hardness than the desired material, or where the material has acquired for one of various reasons accumulations of another material or several other materials of different hardness than the desired material. It may also be the case that the accumulated material is softer than the known surrounding material. The description that is presented here can be easily adjusted such that it is valid also for unaffected material analyse, inspection, where the material is not living tissue.

An arrangement 3 according to the invention can be made to be relatively small. It is currently possible to place a tactile sensor 5 and a Raman spectrometer probe 8 into one and the same arrangement body C. The arrangement body C can be held in one hand. The arrangement of the Raman spectrometer 9 that emits chromatographic light and an arrangement 10 that carries out analysis can also be arranged in the body, or these may be located fully or partially outside of the body itself, being placed in connection with other functions.

It is appropriate that the construction is such that the tactile sensor 5, in the form of a resonance sensor, is arranged around a Raman probe 8, which is then located in the centre. The resonance sensor 5 and the Raman probe 8 should have surfaces 5a and 8a of contact in the same plane such that it will be possible to place the arrangement against the material, and such that it is possible to use both the resonance sensor 5 and the Raman spectrometer 7 during the same occasion of contact.

The body C of the arrangement 3 can have the form of a pen with an extended body 3a that offers a region that can be gripped and held by one hand of the person who is carrying out the analyse. The arrangement should have a part 3b, a point, that can be placed against the material 2 in a firm and clear manner.

It is appropriate that electrical connections, connection arrangements and such, take place in the normal manner using cables or other feed arrangements, or both. The arrangement 3 can be connected in various ways to a computer 11 in which the measured values obtained can be stored, analysed and processed, and compared with other related data.

The arrangement 3, the instrument, is of major benefit during, for example, cancer surgery since a tumour will be well-defined both in terms of its extent and type, and this makes it possible for the surgeons to remove the cancer or tumour completely, without the need to remove quantities of healthy tissue in order to be on the safe side.

There are many applications for an arrangement, an instrument, according to the invention. There is a potential from a medical point of view that large parts of the human body can be analysed, investigated. A gastroscope, which is a long and flexible instrument used to view inside the stomach and gastrointestinal tract, being equipped with this technology would be very powerful and it would be possible to use this gastroscope for many different diagnosis processes and analyses. The method and the arrangement can also be used for the diagnosis of other forms of cancer, for example skin cancer and breast cancer.

## Claims

1. An arrangement (3) that makes possible an unaffected material analysis for the detection and analysis of at least one material deviation in a material (2), comprising a tactile sensor (4) adapted to be placed in contact with the material (2) and adapted to detect at least one region (1) with a difference in hardness in the material (2), an arrangement (7, 8, 9) configured so that when a region with a different hardness is detected by the tactile sensor, it emits chromatographic light (A) to illuminate the detected region (1) with this light in order to obtain reflected light (B) in the form of a light spectrum, and an arrangement (10) configured to analyse the obtained light spectrum and to give information about the material in the region, its physical and chemical molecular structure.

2. An arrangement according to claim 1, in which the tactile sensor (4) is a resonance sensor.

3. An arrangement according to claim 2, in which the resonance sensor (4) comprises an element (5) and an electrical circuit (6) that is activated and that causes the element (5) to vibrate, oscillate, in order to obtain a resonance frequency.

4. An arrangement according to claim 3, in which the element (5) is a piezoelectric element.

5. An arrangement according to any one of claims 1-4, comprising a source (9) of laser light in order to obtain the chromatographic light.

6. An arrangement according to claim 5, comprising a Raman spectrometer (7), which in turn comprises the source (9) of laser light.

7. An arrangement according to any one of claims 1-6, for use in unaffected material analysis of living tissue.

8. An arrangement according to claim 7, for detection and analysis of tumours.

9. An arrangement according to any one of claims 1-8, in which the tactile sensor (4) and at least a part of the arrangement (9) that emits chromatographic light are arranged in a body that can be held by one hand.

10. An arrangement according to claim 9, in which the tactile sensor (4) is arranged around the arrangement (9) that emits chromatographic light, which is then located in the centre of the body.

## Patentansprüche

1. Anordnung (3), welche eine unbeeinflusste Materialanalyse zur Detektion und Analyse zumindest einer Materialabweichung in einem Material (2) ermöglicht,
umfassend
einen Berührungssensor (4), welcher dafür angepasst ist, mit dem Material (2) in Berührung zu kommen, und welcher für die Detektion zumindest eines Bereichs (1) mit einem Härteunterschied des Materials (2) angepasst ist,
eine Anordnung (7, 8, 9), die derart ausgebildet ist, dass sie, wenn ein Bereich mit einer unterschiedlichen Härte vom Berührungssensor detektiert wird, chromatographisches Licht (A) emittiert, um den detektieren Bereich (1) mit diesem Licht zu beleuchten, damit ein reflektiertes Licht (B) in Form eines Lichtspektrums erzielt wird, und
eine Anordnung (10), die dazu ausgebildet ist, das erzielte Lichtspektrum zu analysieren, und Aufschluss über die physikalische und chemische molekulare Struktur des Materials im Bereich zu geben.

2. Anordnung nach Anspruch 1, wobei der Berührungssensor (4) ein Resonanzsensor ist.

3. Anordnung nach Anspruch 2, wobei der Resonanzsensor (4) ein Element (5) und eine elektrische Schaltung (6) umfasst, welche aktiviert wird, und welche eine Vibration, eine Oszillation des Elements (5) verursacht, um eine Resonanzfrequenz zu erzielen.

4. Anordnung nach Anspruch 3, wobei das Element (5) ein piezoelektrisches Element ist.

5. Anordnung nach einem der Ansprüche 1-4, umfassend eine Laserlichtquelle (9) zur Erzielung des chromatographischen Lichts.

6. Anordnung nach Anspruch 5, umfassend ein Raman-Spektrometer (7), das wiederum die Laserlichtquelle (9) umfasst.

7. Anordnung nach einem der Ansprüche 1-6 zur Verwendung bei unbeeinflusster Materialanalyse eines lebenden Gewebes.

8. Anordnung nach Anspruch 7 zur Detektion und Analyse von Tumoren.

9. Anordnung nach einem der Ansprüche 1-8, wobei der Berührungssensor (4) und zumindest ein Teil der das chromatographische Licht emittierenden Anordnung (9) in einem Körper angeordnet sind, der mit einer Hand gehalten werden kann.

10. Anordnung nach Anspruch 9, wobei der Berührungssensor (4) um die Anordnung (9) angeordnet ist, die ein chromatographisches Licht emittiert, das sich dann in der Mitte des Körpers befindet.

## Revendications

1. Dispositif (3) permettant une analyse d'un matériau non affecté pour la détection et l'analyse d'au moins une déviation de matériau dans un matériau (2),
comprenant:
un capteur tactile (4) adapté de manière à être placé en contact avec le matériau (2) et adapté de manière à détecter au moins une région (1) avec une différence de dureté dans le matériau (2),
un dispositif (7, 8, 9) configuré si bien que lorsqu'une région avec une dureté différente est détectée par le capteur tactile, il émet de la lumière chromatographique (A) pour illuminer la région détectée (1) avec cette lumière pour obtenir une lumière réfléchie (B) sous la forme d'un spectre de lumière, et
un dispositif (10) configuré pour l'analyse du spectre de la lumière obtenue pour obtenir des informations concernant le matériau dans la région, sa structure moléculaire physique et chimique.

2. Dispositif selon la revendication 1, dans lequel le capteur tactile (4) est un capteur de résonance.

3. Dispositif selon la revendication 2, dans lequel le capteur de résonance (4) comprend un élément (5) et un circuit électrique (6) qui est activité et qui fait activer l'élément (5) à vibrer, à osciller, afin d'obtenir une fréquence de résonance.

4. Dispositif selon la revendication 3, dans lequel l'élément (5) est un élément piézo-électrique.

5. Dispositif selon l'une quelconque des revendications 1-4, comprenant une source (9) de lumière laser utilisée afin d'obtenir la lumière chromatographique.

6. Dispositif selon la revendication 5, comprenant un spectromètre de Raman (7), qui comprend à son tour la source (9) de lumière laser.

7. Dispositif selon l'une quelconque des revendications 1-6, pour une utilisation dans l'analyse d'un matériau non affecté de tissus vivants.

8. Dispositif selon la revendication 7, pour la détection et l'analyse des tumeurs.

9. Dispositif selon l'une quelconque des revendications 1-8, dans lequel le capteur tactile (4) et au moins une partie du dispositif (9) qui émet la lumière chromatographique sont arrangés dans un corps qui peut être tenu par une seule main.

10. Dispositif selon la revendication 9, dans lequel le capteur tactile (4) est arrangé autour du dispositif (9) qui émet la lumière chromatographique qui peut ensuite être localisée dans le centre du corps.
